# EUROPEAN PATENT SPECIFICATION

(11) **EP 1 802 274 B1**
(45) Date of publication and mention of the grant of the patent: **17.09.2008**
(21) Application number: 05788521.2
(22) Date of filing: 30.09.2005
(51) Int. Cl.: A61K 36/18, A61P 35/04

(54) **USE OF CANNABIDIOL FOR THE INHIBITION OF BRAIN TUMOUR CELL MIGRATION**
VERWENDUNG VON CANNABIDIOL ZUR HEMMUNG VON GEHIRNTUMORZELLMIGRATION
UTILISATION DE CANNABIDIOL POUR INHIBITION DE LA MIGRATION DE CELLULES TUMORALES CEREBRALES

(30) Priority: 01.10.2004 GB 0421900
(43) Date of publication of application: 04.07.2007
(73) Proprietor: GW Pharma Limited, Salisbury, Wiltshire SP4 OJQ (GB)
(72) Inventor: Whittle, Brian, Salisbury, Wiltshire SP4 0JQ (GB); Parolaro, Daniela, DBSF, Pharm. Section, 21052 Busto Arsizio (VA) (IT)
(74) Representative: White, Nina Louise
(86) International application number: PCT/GB2005/003793
(87) International publication number: WO 2006/037981

(56) References cited:
- PORTELLA GIUSEPPE ET AL: "Inhibitory effects of cannabinoid CB1 receptor stimulation on tumor growth and metastatic spreading: actions on signals involved in angiogenesis and metastasis." THE FASEB JOURNAL : OFFICIAL PUBLICATION OF THE FEDERATION OF AMERICAN SOCIETIES FOR EXPERIMENTAL BIOLOGY. SEP 2003, vol. 17, no. 12, September 2003 (2003-09), pages 1771-1773, XP009057753 ISSN: 1530-6860
- LEVY J A ET AL: "Modulation of the metastatic frequency of a murine mammary adenocarcinoma by a snythetic cannabinoid drug" PROCEEDINGS OF THE AMERICAN ASSOCIATION FOR CANCER RESEARCH 1979 UNITED STATES, vol. 20, 1979, page No. 624, XP009057754
- MASSI PAOLA ET AL: "Antitumor effects of cannabidiol, a nonpsychoactive cannabinoid, on human glioma cell lines." THE JOURNAL OF PHARMACOLOGY AND EXPERIMENTAL THERAPEUTICS. MAR 2004, vol. 308, no. 3, March 2004 (2004-03), pages 838-845, XP002357465 ISSN: 0022-3565 cited in the application

## Description

### Field of the invention

The present invention relates to the use of cannabidiol plant extract or a cannabinoid in the manufacture of a medicament for use in the inhibition of central nervous system tumour cell migration.

### Background to the invention

The majority of mortality associated with cancer is due to migration or metastasis of the original tumour cells to sites distant from the initial primary tumour.

The term migration or metastasis is used to describe the process by which cancer cells relocate themselves throughout the body.

The process of migration of tumour cells involves the attachment of the tumour cell to the endothelial basement membrane; this is the thick layer of proteins and glycoproteins that surround tissues. Once attached to the endothelial basement membrane the tumour cell secretes degradative enzymes that are able to break down the proteins in the membrane. The tumour cell is then able to migrate through the body. The tumour cell can enter the bloodstream by squeezing between the cells that make up the blood vessels or they could enter the lymphatic system. When a cancer cell has moved through the blood or lymphatic system to another location it may divide and form a tumour at the new site. This tumour is referred to as a metastatic tumour.

Whether or not cancer cells migrate to other parts of the body depends on many factors including: the type of cancer, the stage of the cancer and the original location of the cancer.

Cancers have been known to affect many areas of the body with the most common types of cancers including: cancer of the bile duct, cancer of the bladder, cancer of the bone, cancer of the bowel (including cancer of the colon and cancer of the rectum), cancer of the brain, cancer of the breast, cancer of the neuroendocrine system (commonly known as a carcinoid), cancer of the cervix, cancer of the eye, cancer of the oesophagus, cancer of the head and neck (this group includes carcinomas that start in the cells that form the lining of the mouth, nose, throat, ear or the surface layer covering the tongue), kaposi's sarcoma, cancer of the kidney, cancer of the larynx, leukaemia, cancer of the liver, cancer of the lung, cancer of the lymph nodes, Hodgkin's lymphoma, non-Hodgkin's lymphoma, melanoma, mesothelioma, myeloma, cancer of the ovary, cancer of the pancreas, cancer of the penis, cancer of the prostate, skin cancer, soft tissue sarcomas, cancer of the spinal cord, cancer of the stomach, testicular cancer, cancer of the thyroid, cancer of the vagina, cancer of the vulva and cancer of the uterus.

A primary brain tumour is a mass created by the growth or uncontrolled proliferation of cells in the brain. A secondary brain tumour is a tumour that has spread to the brain from another part of the body.

A tumour that develops in the brain can destroy or damage brain cells by producing inflammation, compressing other parts of the brain, inducing cerebral oedema (brain swelling) and can cause increases in intracranial pressure (pressure within the skull).

Surgery is the treatment option of choice for many brain tumours, some may be completely excised, but those that are deep or that infiltrate brain tissue may be debulked rather than removed.

Radiation therapy and chemotherapy may be recommended depending on the type of tumour involved.

Glioma cell tumours can often be lethal. The characteristic diffuse infiltrative tumour growth of gliomas often makes the surgical removal of them impossible and this profoundly complicates the clinical management of these patients.

Different approaches are being researched in order to improve the mortality rate of patients diagnosed with a glioma. These include therapies that target the glioma cells but leave normal cells unharmed, methods that limit the spread of the cancer cells and treatments that block the tumours life-sustaining molecules.

One such area of research involves the use of cannabinoids in the inhibition of the viability of cancer cells.

Cannabinoids are the active constituents of cannabis plants and they have been found to demonstrate numerous pharmacological properties.

For example the US patent application US 2004/0039048 (Guzman et al.) describes the treatment of cerebral tumours by the administration of natural or synthetic cannabinoids. It is claimed that activation of specific receptors on the cannabinoids leads to selective death of the transformed cells.

Recently the cannabinoid CBD has been shown to possess anti-tumour properties (Massi et al. J Pharmacol Exp Ther. 2004 Mar; 308(3):838-45). The work described by this paper describes anti-proliferative effects both in-vitro using U87 and U373 human glioma cell lines and in-vivo using U87 human glioma cells subcutaneously implanted to nude mice.

Malignant gliomas are highly infiltrative and proliferative tumours, which follow a characteristic pattern of growth. Glioma cells invade the adjacent normal brain structures and surrounding large blood vessels.

While the use of cannabinoids appear to be useful in the anti-proliferation of tumour cells there is still a significant problem involved in the migration of these tumour cells before they are destroyed.

Inhibition of the migration of glioma cells therefore represents a crucial step in improving the prognosis of patients with malignant gliomas.

The present invention attempts to overcome this problem by the use of a cannabis plant extract or a cannabinoid to impede the progress of cancer cells migrating from their primary tumour location to a secondary site.

### Summary of the invention

According to a first aspect of the present invention there is provided the use of cannabidiol in the manufacture of a medicament for use in the inhibition of central nervous system tumour cell migration.

The invention is useful in a method of inhibiting central nervous system tumour cell migration in a mammalian subject comprising administering to a subject in need thereof an effective amount of cannabidiol.

A preferred embodiment relates to inhibition of central nervous system tumour cell migration in a human patient.

More preferably the cannabidiol is produced from a given cannabis chemovar.

A plant extract is defined as an extract from a plant material as described by the Guidance for Industry Botanical Drug Products Draft Guidance, August 2000, US Department of Health and Human Services, Food and Drug Administration Centre for Drug Evaluation and Research.

Plant material is defined as a plant or plant part (e.g. bark, wood, leaves, stems, roots, flowers, fruits, seeds, berries or parts thereof) as well as exudates.

A chemovar is the term used to describe a hybrid plant that has been propagated to maximise the output of specific chemical constituents. In the case of cannabis chemovars these are often bred to maximise the output of specific cannabinoids.

Preferably the cannabis chemovar expresses said cannabinoid content as one predominant cannabinoid.

The amount of cannabinoid in a certain chemovar can be determined by techniques such as High Pressure Liquid Chromatography (HPLC) or Gas Chromatography (GC). The cannabinoid content of a cannabis chemovar can be described as a percentage of the total dry weight of the cannabis plant material. Alternatively an extract of the cannabis plant material can be produced and the amount of a certain cannabinoid can be expressed as a percentage of the total cannabinoid content.

Certain breeding techniques are able to select cannabis chemovars that produce predominantly one type of cannabinoid. Other chemovars have been produced whereby the cannabis plant produces more than one type of cannabinoid. In certain instances the chemovar can be selected to express two or more cannabinoids in a specific ratio. This can be beneficial as if it is known that a specific ratio of cannabinoids is useful for the treatment of a specific disease or symptom, an extract of the cannabinoids from one type of plant can be prepared rather than producing several extracts and mixing to produce the desired ratio of cannabinoids.

Preferably the cannabis chemovar is selected to predominantly produce one or more of the following cannabinoid(s): tetrahydrocannabinol, delta-9-tetrahydrocannabinol, delta-9-tetrahydrocannabinol propyl analogue, cannabidiol, cannabidiol propyl analogue, cannabinol, cannabichromene, cannabichromene propyl analogue or cannabigerol.

The cannabis chemovar will often produce its cannabinoids in an acid form and these can be decarboxylated into their neutral form after the plant has been harvested. Either the neutral or acid form of the cannabinoid can be suitable for use as described in the present invention.

Preferably the cannabis chemovar is selected to predominantly produce the cannabinoid cannabidiol (CBD).

More preferably the cannabis chemovar has been selected to produce the cannabinoid CBD at an amount greater than or equal to 90% (w/w) of the total amount of cannabinoids in the plant.

Preferably the cannabis plant extract or cannabinoid is in the form of a botanical drug substance.

More preferably the botanical drug substance is prepared using the method described as follows:
i) providing at least one dried Cannabis plant variety for which the amount of cannabinoid is known;
ii) preparing an extract of said at least one Cannabis plant variety using at least one of the following procedures:
   a. maceration
   b. percolation
   c. extraction with solvent such as C₁-C₅ alcohols, norflurane or HFA227
   d. subcritical or supercritical fluid extraction
   e. extraction with hot gas;
iii) formulating a botanical drug substance from said extract or extracts prepared in step (ii) and;
iv) further formulating the botanical drug substance of step (iii) into a pharmaceutical composition with a pharmaceutically acceptable carrier or diluent.

A botanical drug substance is defined as follows. Botanical drug substances which are derived from cannabis plants include primary extracts prepared by such processes as for example, maceration, percolation, extraction with solvents such as C1 to C5 alcohols (e.g. ethanol), Norflurane (HFA134a), HFA227, liquid carbon dioxide under pressure and extraction using a hot gas. The primary extract may be further purified by supercritical or subcritical extraction, vaporisation and chromatography. When solvents such as those listed above are used the resultant extract may contain non-specific lipid-soluble material. This can be removed by a variety of processes including winterisation, which involves chilling to -20°C followed by filtration to remove waxy ballast, extraction with liquid carbon dioxide and by distillation.

Preferred cannabis extracts include those which are obtainable by using any of the methods or processes disclosed herein or in UK patent number GB2380129, the contents of which are incorporated herein in their entirety by reference. The extracts are preferably free of waxes and other non-specific lipid soluble material but preferably contain substantially all of the cannabinoids naturally present in the plant.

Botanical drug substances are formulated into Botanical Drug Products which are defined in the Guidance for Industry Botanical Drug Products Draft Guidance, August 2000, US Department of Health and Human Services, Food and Drug Administration Centre for Drug Evaluation and Research as: "A botanical product that is intended for use as a drug; a drug product that is prepared from a botanical drug substance."

The type of tumour cell that is prevented from migration is a central nervous system tumour cell, preferably a brain tumour cell.

More preferably the type of tumour cell that is prevented from migration is a glioma tumour cell.

Brain tumours are usually classified according to the location of the tumour and the type of cell that the cancer has developed from.

For example different types of brain tumour include: acoustic neuroma, astrocytoma, CNS lymphoma, ependymoma, haemangioblastoma, medulloblastoma, meningioma, glioma, mixed glioma, oligodendroglioma, pineal region tumours and pituitary tumours.

Gliomas are tumours of the glial cells; these cells support and protect nerve cells in the brain. Gliomas comprise nearly half of all primary brain tumours and a fifth of all primary spinal cord tumours.

Certain aspects of this invention are further described, by way of example only, with reference to the accompanying drawings in which:
Figure 1 is a graph detailing the concentration dependant inhibition of U87 human glioma cells induced by CBD. Results are expressed as a percentage of migration versus control.
Figure 2 is a graph detailing that CBD-induced cell migration is not prevented by pre-treatment with the selective antagonists for CB1 (SR141716) or CB2 (SR144528) receptors.

### Specific description

The effect of the cannabinoid cannabidiol (CBD) was investigated in its ability to modulate the motility of human glioma cells. The features of the invention are illustrated further by reference to the following nonlimiting example:

### Example 1:

Cannabidiol (CBD) in the form of a botanical drug substance was dissolved in ethanol to a concentration of 100mM this was stored at -20°C until required.

Before use the CBD was further diluted with tissue culture medium to the desired concentration, ensuring that the concentration of ethanol was below 0.001%.

U87 human glioma cells were used throughout this experiment. The cells were maintained at 37°C in a humidified atmosphere with 5% CO₂ and 95% air.

Cells were cultured in a 75cm² culture flask in Dulbecco's Modified Eagle Medium (DMEM), which had been supplemented with 4mM L-glutamine, 100 units/ml penicillin, 100 mg/ml streptomycin, 1% sodium pyruvate, 1% non-essential amino acids and 10% heat-inactivated fetal bovine serum.

A cell migration assay was undertaken with the U87 cells in a 48 well, modified Boyden chamber in which upper and lower compartments were separated by a polycarbonate filter with a pore diameter of 8µm coated with 15µg/ml of fibronectin. Conditioned medium (CM) served as a chemoattractant. The CM was made by incubating a subconfluent culture of U87 cells with complete medium for 3 days.

The coated filter was placed over the bottom chamber, which contained the conditioned medium. Serum-free medium was used as a negative control.

U.87 cells were treated with either CBD or vehicle for 30 minutes and then seeded in the upper chamber at a concentration of 3 x 10⁴ cells per well and incubated for 6 hours at 37°C.

After the incubation the non-migrated cells on the upper surface of the filter were removed by scraping and the migrated cells on the lower side of the filter were stained with Diff-Quick Stain. Between 5 and 8 unit fields per filter were counted at 400x magnification.

### Results:

The addition of CBD to the culture medium of human glioma cells U87 resulted in a concentration-dependant inhibition of migration.

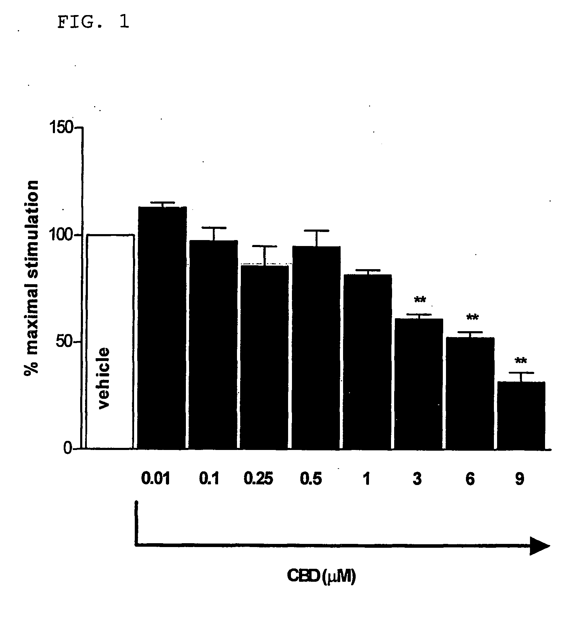

As is shown in Figure 1 cells were exposed to an increased concentration of CBD in a range starting from 0.01µM to 9µM and their migration was evaluated after 6 hours. The degree of inhibition of cell migration is expressed as a percentage of inhibition versus vehicle treatment (maximal stimulation).

The IC₅₀ of CBD was determined to be 5.05 ± 1.1µM. The range of concentrations of CBD that were used did not alter cell viability.

### Example 2:

Much of the data generated in research on cannabinoids has shown that their pharmacological effects on the central nervous system are mediated by cannabinoid receptors.

In order to determine whether the CBD-induced inhibition of cell migration as described in Example 1 above was dependant on the stimulation of these receptors the cell migration assay was performed with specific antagonists selective to CB1 and CB2 receptors. These are SR141716A and SR144528 respectively.

The presence of the CB1 and CB2 receptors in U87 human glioma cells was firstly checked by immunoblot experiments and both receptors were found to be present, (data not shown).

U87 human glioma cells were firstly pre-treated with the antagonists for 30 minutes and then treated with the CBD for an additional 30 minutes before seeding in the upper chamber of the Boyden chamber.

The cell migration assay was performed using a CBD concentration of 6µM (shown in Example 1 to inhibit 50% migration. The CB1 and CB2 receptor antagonists were tested at concentrations of 0.1µM and 1 µM, at these concentrations cell viability was not affected.

### Results:

Pre-treatment of the U87 human glioma cells with CB1 and CB2 receptor antagonists had little effect in the CBD-induced inhibition of migration.

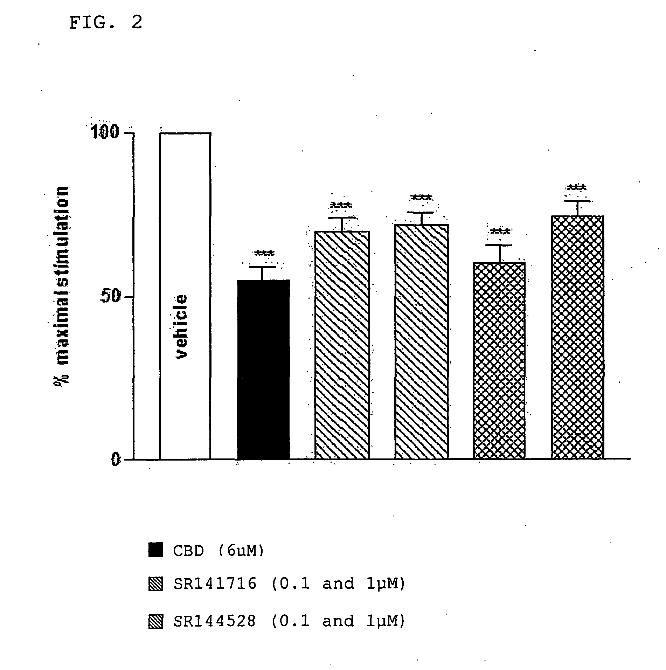

As shown in figure 2, it is indicated that the CBD-induced effect was not mediated by the classical cannabinoid receptors.

## Claims

1. Use of cannabidiol in the manufacture of a medicament for use in the inhibition of brain tumour cell migration.

2. Use of cannabidiol in the manufacture of a medicament for use in the inhibition of central nervous system tumour cell migration.

3. Use of cannabidiol in the manufacture of a medicament for use in the inhibition of glioma tumour cell migration.

4. Use as claimed in any one of claims 1 to 3, wherein the cannabidiol is produced from a given cannabis chemovar.

5. Use as claimed in claim 4, wherein the cannabidiol is produced from the cannabis chemovar at an amount greater than or equal to 90% (w/w) of the total amount of cannabinoids in the plant.

6. Use as claimed in any of the preceding claims, wherein the cannabidiol is in the form of a botanical drug substance.

## Patentansprüche

1. Verwendung von Cannabidiol zur Herstellung eines Medikaments zur Verwendung bei der Hemmung von Gehirntumorzellmigration.

2. Verwendung von Cannabidiol zur Herstellung eines Medikaments zur Verwendung bei der Hemmung der Migration von Tumorzellen des zentralen Nervensystems.

3. Verwendung von Cannabidiol zur Herstellung eines Medikaments zur Verwendung bei der Hemmung von Gliomtumorzellmigration.

4. Verwendung wie in einem der Ansprüche 1 bis 3 beansprucht, worin das Cannabidiol von einem bestimmten Cannabis-Chemovar produziert wird.

5. Verwendung wie in Anspruch 4 beansprucht, worin das Cannabidiol von dem Cannabis-Chemovar in einer Menge, die größer oder gleich 90 Gewichts-% der Gesamtmenge an Cannabinoiden in der Pflanze ist, produziert wird.

6. Verwendung wie in einem der vorhergehenden Ansprüche beansprucht, worin das Cannabidiol in Form einer pflanzlichen Arzneimittelsubstanz ist.

## Revendications

1. Utilisation de cannabidiol dans la production d'un médicament destiné à être utilisé dans l'inhibition de la migration de cellules tumorales cérébrales.

2. Utilisation de cannabidiol dans la production d'un médicament destiné à être utilisé dans l'inhibition de la migration de cellules tumorales du système nerveux central.

3. Utilisation de cannabidiol dans la production d'un médicament destiné à être utilisé dans l'inhibition de la migration de cellules tumorales de gliome.

4. Utilisation suivant l'une quelconque des revendications 1 à 3, dans laquelle le cannabidiol est produit à partir d'un chimiovar de cannabis donné.

5. Utilisation suivant la revendication 4, dans laquelle le cannabidiol est produit à partir du chimiovar de cannabis en une quantité supérieure ou égale à 90 % (en poids/poids) de la quantité totale de cannabinoïdes dans la plante.

6. Utilisation suivant l'une quelconque des revendications précédentes, dans laquelle le cannabidiol est sous forme d'une substance médicamenteuse végétale.
